# EUROPEAN PATENT SPECIFICATION

(11) **EP 3 612 626 B1**
(45) Date of publication and mention of the grant of the patent: **03.12.2025**
(21) Application number: 18721585.0
(22) Date of filing: 11.04.2018
(51) Int. Cl.: C12N 9/00, C12P 7/10

(54) **SELECTED ACETYL-COA SYNTHASE ENZYMES FOR REDUCTION OF ACETATE PRODUCTION IN YEAST**
AUSGEWÄHLTE ACETYL-COA-SYNTHASEENZYME ZUR VERRINGERUNG DER ACETATPRODUKTION IN HEFE
ENZYMES ACÉTYL-COA SYNTHÉTASE SÉLECTIONNÉES POUR LA RÉDUCTION DE LA PRODUCTION D'ACÉTATE DANS LA LEVURE

(30) Priority: 17.04.2017 US 201762486178 P
(43) Date of publication of application: 26.02.2020
(73) Proprietor: Danisco US Inc., Palo Alto, California 94304 (US)
(72) Inventor: MIASNIKOV, Andrei, Union City, California 94587 (US)
(74) Representative: Mewburn Ellis LLP
(86) International application number: PCT/US2018/027100
(87) International publication number: WO 2018/194892

(56) References cited:
- WO-A1-2011/149353
- WO-A1-2016/188813
- WO-A1-2017/189421
- JUN DING ET AL: "Overexpression of acetyl-CoA synthetase in Saccharomyces cerevisiae increases acetic acid tolerance", FEMS MICROBIOLOGY LETTERS, vol. 362, no. 3, 1 February 2015 (2015-02-01), pages 1 - 7, XP055380703, DOI: 10.1093/femsle/fnu042
- DE JONG-GUBBELS PATRICIA ET AL: "Overproduction of acetyl-coenzyme A synthetase isoenzymes in respiring Saccharomyces cerevisiae cells does not reduce acetate production after exposure to glucose excess", FEMS MICROBIOLOGY LETTERS, WILEY-BLACKWELL PUBLISHING LTD, GB, vol. 165, no. 1, 1 August 1998 (1998-08-01), pages 15 - 20, XP009506984, ISSN: 0378-1097, DOI: 10.1111/J.1574-6968.1998.TB13121.X
- M A VAN DEN BERG ET AL: "The two acetyl-coenzyme A synthetases of Saccharomyces cerevisiae differ with respect to kinetic properties and transcriptional regulation.", JOURNAL OF BIOLOGICAL CHEMISTRY, vol. 271, no. 46, 1 November 1996 (1996-11-01), pages 28953 - 28959, XP055089309, ISSN: 0021-9258, DOI: 10.1074/jbc.271.46.28953
- M S JETTEN ET AL: "Isolation and characterization of acetyl-coenzyme A synthetase from Methanothrix soehngenii.", JOURNAL OF BACTERIOLOGY, vol. 171, no. 10, 1 October 1989 (1989-10-01), US, pages 5430 - 5435, XP055494615, ISSN: 0021-9193, DOI: 10.1128/jb.171.10.5430-5435.1989
- DATABASE UniProt [online] 28 June 2011 (2011-06-28), "RecName: Full=Acetyl-coenzyme A synthetase {ECO:0000256|HAMAP-Rule:MF_01123}; Short=AcCoA synthetase {ECO:0000256|HAMAP-Rule:MF_01123}; Short=Acs {ECO:0000256|HAMAP-Rule:MF_01123}; EC=6.2.1.1 {ECO:0000256|HAMAP-Rule:MF_01123}; AltName: Full=Acetate--CoA ligase {ECO:0000256|HAMAP-Rule:MF_01123}; AltN", XP055494612, retrieved from EBI accession no. UNIPROT:F4BX08 Database accession no. F4BX08

## Description

### TECHNICAL FIELD

The present compositions and methods relate to yeast having reduced acetate production as a consequence of expressing selected acetyl-Co synthase (ACS) enzymes. Such yeast may also be modified for reduced glycerol and/or increased ethanol production. The yeast is useful for producing ethanol from carbohydrate-containing substrates.

### BACKGROUND

Yeast-based ethanol production is based on the conversion of sugars into ethanol. The current annual fuel ethanol production by this method is about 90 billion liters worldwide. It is estimated that about 70% of the cost of ethanol production is the feedstock. Since the ethanol production volume is so large, even small yield improvements have massive economic impact for the industry. The conversion of one mole of glucose into two moles of ethanol and two moles of carbon dioxide is redox-neutral, with the maximum theoretical yield being about 51%. The current industrial yield is about 45%; therefore, there are opportunities to increase ethanol production.

Carbon dioxide, glycerol and yeast biomass are the major by-products of ethanol fermentation. During yeast growth and fermentation, a surplus of NADH is generated, which is used to produce glycerol for the purposes of redox balance and osmotic protection. Glycerol is considered a low value product and several approaches have been taken to reduce glycerol production.

Engineered yeast cells having a heterologous phosphoketolase pathway have been previously described (*e.g*., WO2015148272). These cells express heterologous phosphoketolase (PKL; EC 4.1.2.9) and phosphotransacetylase (PTA; EC 2.3.1.8), optionally with other enzymes, to channel carbon flux away from the glycerol pathway and toward the synthesis of acetyl-coA, which is then converted to ethanol. These cells are capable of increased ethanol production in a fermentation process when compared to otherwise-identical parent yeast cells. Unfortunately, such modified also produce increased acetate.

Acetate is toxic to most microorganisms including yeast. Wild-type yeast produces only minor amounts of acetate (*e.g.,* 100-300 mg/l in a medium containing 60 g/l glucose). Acetate production in yeast expressing PKL is greatly increased (*e.g*., 1-2g/l in the same medium). Under conditions of industrial grain ethanol production where total amount of fermented glucose reaches 300 g/l, acetate production by PKL-expressing yeast strains can be extrapolated to 5-10 g/l. In reality, such concentrations are not observed because the cells stop growing due to acetate poisoning.

Several researchers have tried to over-express acetyl-Co synthase (ACS) to reduce acetate accumulation in PKL-expressing yeast. For, example, baker's yeast contains two ACS isoenzymes ACS1 and ACS2. ACS1 is under tight post-translational control being subject to catabolite inactivation in the presence of glucose (De Jong-Gubbels, P. et al. (1997) FEMS Microbiology Letters 153:75-81) while ACS2 has about 30-fold higher Km for acetate than ACS1 (van den Berg et al. (1996) J. Biol. Chem. 271:28953-59). In a study specifically aiming at reducing acetate production in yeast, de Jong-Gubbels et al. concluded that over-expression of either of the two native Saccharomyces cerevisiae ACS fails to decrease acetate level in yeast culture media ((1998) FEMS Microbiology Letters 165:15-20). In later, applied studies, attempts were made to use either native ACSI enzyme or a mutant (L614P) variant of *Salmonella. typimurium* ACS that was reported to be resistant to catabolite inactivation. While the *S. typhimurium* enzyme preformed somewhat better than ACSI, acetate reduction was only modest.

In addition to being produced by certain yeast, some feedstocks used for the production of ethanol naturally contain acetate, which has the same detrimental effect on yeast. Accordingly, for various reasons, the need exists to modify yeast metabolic pathways to maximize ethanol production, while not increasing the production of undesirable pathway by-products such as acetate.

WO2016/188813 and WO2011/149353 relate to ethanol producing yeast cells. Ding et al. FEMS Microbiology Letters, 362 (2015) 1-7, de Jong-Gubbels et al. FEMS Microbiology Letters 165 (1998) 15-20, Van den Berg et al. Journal of Biological Chemistry 271, 46, 1 Nov 1996 28953-28959 and Jetten et al. Journal of Bacteriology 171, 10 1 October 1989, 5430-5435 discuss acetyl-CoA synthetase in yeast.

### SUMMARY

The present compositions and methods relate to yeast exhibiting reduced acetate production due to the expression of selected acetyl-Co synthase (ACS) enzymes. The modified yeast cells and method for reducing the amount of acetate produced by yeast cells are defined in the claims. The invention includes modified yeast cells comprising an exogenous gene encoding a protein having at least 80% amino acid sequence identity to a polypeptide of SEQ ID NO: 10, or a fragment thereof, the protein and fragment thereof having acetyl CoA synthetase (ACS) activity, wherein the modified cells produce a reduced amount of acetate and/or grow in the presence of an increased amount of acetate, compared to otherwise identical yeast cells not comprising the exogenous gene.

In some embodiments the modified yeast cells further comprising a genetic modification that, in the absence of the exogenous gene, causes the yeast cells to produce an increased amount of acetate. The genetic modification may be the introduction of one or more genes of the phosphoketolase pathway or the deletion or disruption of genes of the glycerol synthesis pathway.

In some embodiments of the modified yeast cells the reduction in the amount of acetate produced is at least 20%, at least 30% or at least 40%.

In some embodiments of the modified yeast cells the protein has at least 90%, at least 95% or at least 97% amino acid sequence identity to a polypeptide of SEQ ID NO: 10, or a fragment thereof, the protein and fragment thereof having ACS activity.

In some embodiments the modified yeast cells further comprise a gene encoding a carbohydrate processing enzyme or other protein of interest.

The invention includes a method for reducing the amount of acetate produced by yeast cells during a fermentation of a starch hydrolysate, comprising, introducing into the yeast an exogenous gene encoding a protein having at least 80% amino acid sequence identity to a polypeptide of SEQ ID NO: 10, or a fragment thereof, the protein and fragment thereof having ACS activity, in order to produce the modified yeast cells of the invention, and using the modified yeast cells in the fermentation of a starch hydrolysate.

In some embodiments of the method the yeast cells further comprise a genetic modification that, in the absence of the exogenous gene, would cause the yeast cells to produce an increased amount of acetate. The genetic modification may be the introduction of genes of the phosphoketolase pathway or the deletion or disruption of genes of the glycerol synthesis pathway.

In some embodiments of the method the reduction in the amount of acetate produced is at least 20%, at least 30% or at least 40%.

In some embodiments of the method the protein has at least 90%, at least 95% or at least 97% amino acid sequence identity to a polypeptide of SEQ ID NO: 10, or a fragment thereof, the protein and fragment thereof having ACS activity.

### BRIEF DESCRIPTION OF THE DRAWINGS

Figure 1 is a functional map of vector pX(DeltaTDH_ACSSalty), the prototype for all constructs used to express various acetyl-CoA synthases described, herein.
Figure 2 is a functional map of vector pPATH1(TDH_A2_ADE2). This vector carries three expression cassettes for phosphoketolase from *Bifidobacterium animalis,* phosphotransacetylase from *Lactobacillus plantarum* and acylating acetaldehyde dehydrogenase from *Salmonella enterica.* Together these three enzymes form a metabolic pathway referred to as the "phosphoketolase pathway."
Figure 3 is a graph showing reduced acetate production in derivatives of high-acetate-producing-strain A2 expressing various homologous and heterologous ACS enzymes. Strain FERMAXGOLD^{®} is the parental strain of A2 and produces acetate in amounts typical for wild-type yeast strains.

### DETAILED DESCRIPTION

### I. Definitions

Prior to describing the present compositions and methods in detail, the following terms are defined for clarity. Terms not defined should be accorded their ordinary meanings as used in the relevant art.

As used herein, the term "gene" is synonymous with the term "allele" in referring to a nucleic acid that encodes and directs the expression of a protein or RNA.

As used herein, the terms "wild-type" and "native" are used interchangeably and refer to genes, proteins, strains, and biochemical pathways found in nature.

As used herein, "deletion of a gene," refers to its removal from the genome of a host cell. Deletion may be complete, meaning an entire gene (*i.e.,* at least the entire coding sequences are removed) or partial, meaning that only a portion of the coding sequences or regulatory sequences are removed but which prevent the production of a functional gene product.

As used herein, "attenuation of a pathway" or "attenuation of the flux through a pathway" *i.e.,* a biochemical pathway, refers broadly to any genetic or chemical manipulation that reduces or completely stops the flux of biochemical substrates or intermediates through a metabolic pathway. Attenuation of a pathway may be achieved by a variety of well-known methods. Such methods include but are not limited to: complete or partial deletion of one or more genes, replacing wild-type alleles of these genes with mutant forms encoding enzymes with reduced catalytic activity or increased Km values, modifying the promoters or other regulatory elements that control the expression of one or more genes, engineering the enzymes or the mRNA encoding these enzymes for a decreased stability, misdirecting enzymes to cellular compartments where they are less likely to interact with substrate and intermediates, the use of interfering RNA, and the like.

As used herein, "disruption of a gene" refers broadly to any genetic manipulation that substantially prevents a cell from producing a functional gene product. Exemplary methods of gene disruption include complete or partial deletion of a gene and making mutations in coding or regulatory sequences.

As used herein, the terms "genetic manipulation" and "genetic alteration" are used interchangeably and refer to the alteration/change of a nucleic acid sequence. The alteration can be included but is not limited to a substitution, deletion, insertion or chemical modification of at least one nucleic acid in the nucleic acid sequence.

As used herein, "expressing a polypeptide" and similar terms, refer to the cellular process of producing a polypeptide using the translation machinery (*e.g*., ribosomes) of the cell.

As used herein, "overexpressing a polypeptide," "increasing the expression of a polypeptide," and similar terms, refer to expressing a polypeptide at higher-than-normal levels compared to those observed with parental or "wild-type cells that do not include a specified genetic modification. Overexpression can be accomplished by using a stronger promoter with an endogenous gene and/or introducing additional copies of a gene into a cell, *e.g.,* in the form of an additional expression cassette, using a suitable promoter.

As used herein, an "expression cassette" refers to a nucleic acid that includes an amino acid coding sequence, promoters, terminators, and other nucleic acid sequence needed to allow the encoded polypeptide to be produced in a cell. Expression cassettes can be exogenous (*i.e.,* introduced into a cell) or endogenous (*i.e.,* extant in a cell).

As used herein, "anaerobic fermentation" refers to growth in the absence of oxygen.

As used herein, "aerobic fermentation" refers to growth in the presence of oxygen.

As used herein, the terms "polypeptide" and "protein" (and/or their respective plural forms) are used interchangeably to refer to polymers of any length comprising amino acid residues linked by peptide bonds. The conventional one-letter or three-letter codes for amino acid residues are used herein. The polymer can be linear or branched, it can comprise modified amino acids, and it can be interrupted by non-amino acids. The terms also encompass an amino acid polymer that has been modified naturally or by intervention; for example, disulfide bond formation, glycosylation, lipidation, acetylation, phosphorylation, or any other manipulation or modification, such as conjugation with a labeling component. Also included within the definition are, for example, polypeptides containing one or more analogs of an amino acid (including, for example, unnatural amino acids, etc.), as well as other modifications known in the art.

As used herein, functionally and/or structurally similar proteins are considered to be "related proteins." Such proteins can be derived from organisms of different genera and/or species, or even different classes of organisms (*e.g*., bacteria and fungi). Related proteins also encompass homologs determined by primary sequence analysis, determined by secondary or tertiary structure analysis, or determined by immunological cross-reactivity.

As used herein, the term "percent amino acid sequence identity," or similar, means that a particular sequence has at least a certain percentage of amino acid residues identical to those in a specified reference sequence, when aligned using the CLUSTAL W algorithm with default parameters. *See* Thompson et al. (1994) Nucleic Acids Res. 22:4673-80. Default parameters for the CLUSTAL W algorithm are:

| | |
|---|---|
| Gap opening penalty: | 10.0 |
| Gap extension penalty: | 0.05 |
| Protein weight matrix: | BLOSUM series |
| DNA weight matrix: | IUB |
| Delay divergent sequences %: | 40 |
| Gap separation distance: | 8 |
| DNA transitions weight: | 0.50 |
| List hydrophilic residues: | GPSNDQEKR |
| Use negative matrix: | OFF |
| Toggle Residue specific penalties: | ON |
| Toggle hydrophilic penalties: | ON |
| Toggle end gap separation penalty | OFF |

As used herein, the singular articles "a," "an," and "the" encompass the plural referents unless the context clearly dictates otherwise. The following abbreviations/acronyms have the following meanings unless otherwise specified:
- EC: enzyme commission
- kDa: kiloDalton
- kb: kilobase
- MW: molecular weight
- w/v: weight/volume
- w/w: weight/weight
- v/v: volume/volume
- wt%: weight percent
- °C: degrees Centigrade
- H₂O: water
- H₂O₂: hydrogen peroxide
- dH₂O or DI: deionized water
- dIH₂O: deionized water, Milli-Q filtration
- g or gm: gram
- µg: microgram
- mg: milligram
- kg: kilogram
- lb: pound
- µL and µl: microliter
- mL and ml: milliliter
- mm: millimeter
- µm: micrometer
- mol: mole
- mmol: millimole
- M: molar
- mM: millimolar
- µM: micromolar
- nm: nanometer
- U: unit
- ppm: parts per million
- sec and ": second
- min and ': minute
- hr and h: hour
- EtOH: ethanol
- eq.: equivalent
- PCR: polymerase chain reaction
- DNA: deoxyribonucleic acid
- Δ: relating to a deletion
- bp: base pairs

### II. Yeast expressing selected acetyl-Co synthase enzymes

The present compositions and methods relate to the use of selected acetyl-Co synthase (ACS) enzymes to reduce the production of acetate in engineered yeast cells. The selected ACS were identified after screening a number of different taxonomically-divergent ACS. Several ACS outperformed any reported in the literature. An archaeal ACS from *Methanosaeta concilii* ACS was the overall winner while a low-affinity ACS from yeast *Saccharomyces cerevisiae* and *Zygosaccharomyces rouxii* (ACS2) also performed better than the most efficient ACS reported previously. The decrease in acetate production in yeast resulting from the introduction of an exogenous gene encoding one of these enzymes (or overexpressing an endogenous gene) is at least 10%, at least 20%, at least 30% and even at least 40%, compared to the amount of acetate produced by yeast that over-produces acetate.

Use of the selected acetyl-Co synthase (ACS) enzymes will reduce the production of acetate in yeast that produce an unwanted excess of acetate, including but not limited to engineered yeast such as those described by Miasnikov (WO2015148272) and Pronk et al. (WO2011010923), and/or reduce the amount of exogenous acetate naturally present in the fermentation medium.

### III. Combination with additional mutations that affect alcohol production

In some embodiments, not specifically claimed, the present modified cells may additionally express heterologous phosphoketolase (PKL; EC 4.1.2.9) and phosphotransacetylase (PTA; EC 2.3.1.8), optionally with other enzymes, to channel carbon flux away from the glycerol pathway and toward the synthesis of acetyl-coA, which is then converted to ethanol. An exemplary phosphoketolase can be obtained from *Gardnerella vaginalis* (UniProt/TrEMBL Accession No.: WP_016786789). An exemplary phosphotransacetylase can be obtained from *Lactobacillus plantarum* (UniProt/TrEMBL Accession No.: WP_003641060). In some embodiments the present modified cells may further have an artificial alternative pathway for making Ac-CoA that does not contribute to a redox cofactor imbalance in the cells under anaerobic conditions, as described by Miasnikov (WO2015148272).

The present modified cells may further include mutations that result in attenuation of the native glycerol biosynthesis pathway, which are known to increase alcohol production. Methods for attenuation of the glycerol biosynthesis pathway in yeast are known and include reduction or elimination of endogenous NAD-dependent glycerol 3-phosphate dehydrogenase (GPD) or glycerol phosphate phosphatase activity (GPP), for example by disruption of one or more of the genes *GPD1, GPD2, GPP1* and/or *GPP2.* See, *e.g.,* U.S. Patent Nos. 9,175,270 (Elke et al.), 8,795,998 (Pronk et al.) and 8,956,851 (Argyros et al.)*.*

In some embodiments the present modified cells may further include a heterologous gene encoding a protein with NAD+-dependent acetylating acetaldehyde dehydrogenase activity and/or a heterologous gene encoding a pyruvate-formate lyase. The introduction of such genes in combination with attenuation of the glycerol pathway is described, *e.g*., in U.S. Patent No. 8,795,998 (Pronk et al.)*.* However, in most embodiments of the present compositions and methods, the introduction of an acetylating acetaldehyde dehydrogenase and/or a pyruvate-formate lyase is not required because the need for these activities is obviated by the attenuation of the native biosynthetic pathway for making acetyl-CoA that contributes to redox cofactor imbalance.

In some embodiments the present modified cells may further have an attenuated native biosynthetic pathway in yeast for making Ac-CoA, which contributes to redox cofactor imbalance in the cells under anaerobic conditions and thus requires glycerol production for restoring the redox balance. In some embodiments, the compositions and methods involve disruption of one, several or all the native genes (*e.g., ALD*2 *ALD*3 *ALD*4 *ALD*5 and *ALD*6) encoding aldehyde dehydrogenase (EC 1.2.1.3). The native yeast Ac-CoA pathway, including aldehyde dehydrogenase, is well described in the literature. Deletion of these native genes has been described in, *e.g.,* Kozak et al. (2014) Metabolic Engineering 21:46-59).

In some embodiments, the present modified yeast cells further comprise a butanol biosynthetic pathway. In some embodiments, the butanol biosynthetic pathway is an isobutanol biosynthetic pathway. In some embodiments, the isobutanol biosynthetic pathway comprises a polynucleotide encoding a polypeptide that catalyzes a substrate to product conversion selected from the group consisting of: (a) pyruvate to acetolactate; (b) acetolactate to 2,3-dihydroxyisovalerate; (c) 2,3-dihydroxyisovalerate to 2-ketoisovalerate; (d) 2-ketoisovalerate to isobutyraldehyde; and (e) isobutyraldehyde to isobutanol. In some embodiments, the isobutanol biosynthetic pathway comprises polynucleotides encoding polypeptides having acetolactate synthase, keto acid reductoisomerase, dihydroxy acid dehydratase, ketoisovalerate decarboxylase, and alcohol dehydrogenase activity.

In some embodiments, the modified yeast cells comprising a butanol biosynthetic pathway further comprise a modification in a polynucleotide encoding a polypeptide having pyruvate decarboxylase activity. In some embodiments, the yeast cells comprise a deletion, mutation, and/or substitution in an endogenous polynucleotide encoding a polypeptide having pyruvate decarboxylase activity. In some embodiments, the polypeptide having pyruvate decarboxylase activity is selected from the group consisting of: PDC1, PDC5, PDC6, and combinations thereof. In some embodiments, the yeast cells further comprise a deletion, mutation, and/or substitution in one or more endogenous polynucleotides encoding FRA2, ALD6, ADH1, GPD2, BDH1, and YMR226C.

In some embodiments, the present modified cells include any number of additional genes of interest encoding proteins of interest in addition to the ACS enzyme. Proteins of interest, include selectable markers, carbohydrate-processing enzymes, and other commercially-relevant polypeptides, including but not limited to an enzyme selected from the group consisting of a dehydrogenase, a transketolase, a phosphoketolase, a transladolase, an epimerase, a phytase, a xylanase, a β-glucanase, a phosphatase, a protease, an α-amylase, a β-amylase, a glucoamylase, a pullulanase, an isoamylase, a cellulase, a trehalase, a lipase, a pectinase, a polyesterase, a cutinase, an oxidase, a transferase, a reductase, a hemicellulase, a mannanase, an esterase, an isomerase, a pectinases, a lactase, a peroxidase and a laccase. Proteins of interest may be secreted, glycosylated, and otherwise modified.

### IV. Use of the modified yeast for increased alcohol production

The present compositions and methods include methods for increasing alcohol production using the modified yeast in fermentation reactions. Such methods are not limited to a particular fermentation process. The present engineered yeast is expected to be a "drop-in" replacement for convention yeast in any alcohol fermentation facility. While primarily intended for fuel ethanol production, the present yeast can also be used for the production of potable alcohol, including wine and beer.

### V. Yeast cells suitable for modification

Yeasts are unicellular eukaryotic microorganisms classified as members of the fungus kingdom and include organisms from the phyla Ascomycota and Basidiomycota. Yeast that can be used for alcohol production include, but are not limited to, *Saccharomyces* spp., including *S*. *cerevisiae,* as well as *Kluyveromyces, Lachancea* and *Schizosaccharomyces* spp. Numerous yeast strains are commercially available, many of which have been selected or genetically engineered for desired characteristics, such as high alcohol production, rapid growth rate, and the like. Some yeasts have been genetically engineered to produce heterologous enzymes, such as glucoamylase or α-amylase.

### VI. Carbohydrate substrates and processes

Ethanol production from a number of carbohydrate substrates is well known, as are innumerable variations and improvements to enzymatic and chemical conditions and mechanical processes. The present compositions and methods are believed to be fully compatible with such substrates and conditions.

### EXAMPLES

### Example 1. Construction of expression vectors for testing acetyl-CoA synthases

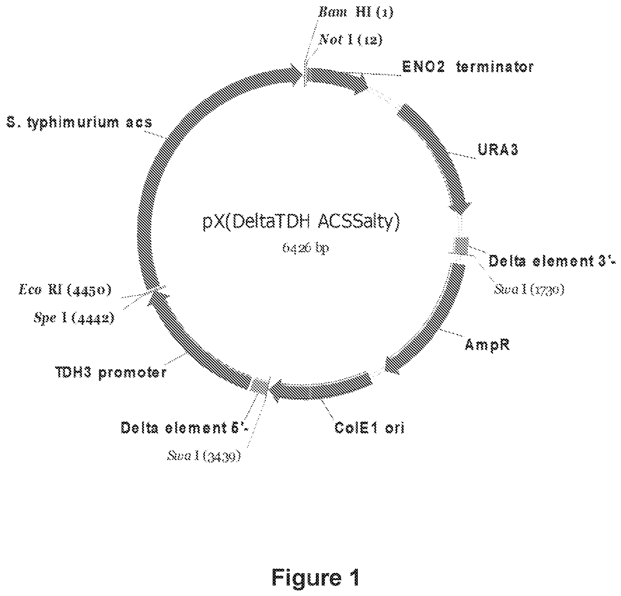

The coding regions of the *S. cerevisiae* genes *ACS*1 and *ACS*2 as well as *Z*. *rouxii* gene *ACS2* were amplified by PCR using chromosomal DNA of the corresponding hosts as templates. The genes encoding the *Salmonella typhimurium acs* mutant L641P and the gene encoding the *Methanosaeta concilii* acsA1 were synthesized based on known amino acid sequence. The codon composition of the synthetic genes was optimized to correspond to the codon bias of the highly-expressed genes of *S. cerevisiae.* Nucleotide and amino acid sequences of all the genes encoding various ACS of this study are listed as SEQ ID NO: 1-10. Vector pX(DeltaTDH_ACSSalty) (Figure 1) was assembled using conventional genetic engineering methods. All yeast-derived elements in this vector, *i.e.,* the *THD*3 promoter and *ENO*2 transcription terminator, 3' and 5' sequences of the yeast δ-element, and the *URA*3 selectable marker, were derived from yeast chromosomal DNA. Bacterial sequences (*i.e.,* the colE1 replication origin and ampicillin resistance gene) were derived from the well-known pUC19 vector. The vectors for expression of other ACSs were identical to pX(DeltaTDH_ACSSalty) except that the coding sequence of *S. typhimurium* ACS L641P variant was replaced with the sequences of SEQ ID NO: 1, 3, 5 or 9.

### Example 2. Testing the effect of different ACS on acetate production in yeast

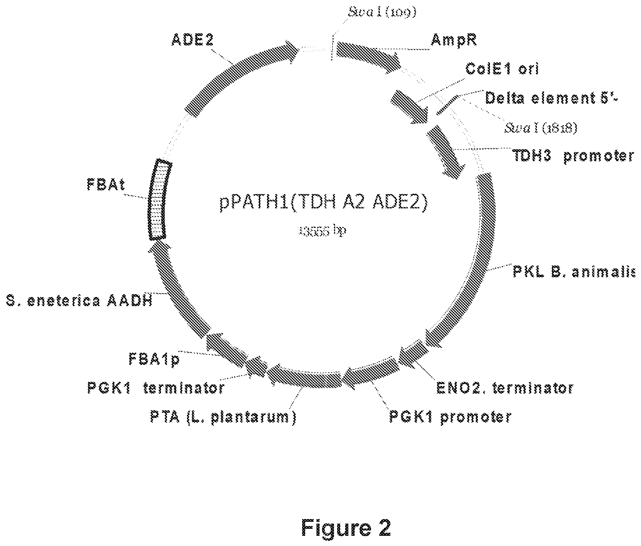

For testing the ability of different ACS to help yeast assimilate excessive acetate, a test strain of yeast that over-produces acetate was used. This strain, herein referred to as A2, was obtained by transforming a *ura*3, *ade*2 mutant of the commercially-available *S. cerevisiae* strain FERMAXGOLD^{®} with the large *Swa*I fragment of vector PATH1(TDH_A2_ADE2) (Figure 2). Strain A2 expresses three enzymes: phosphoketolase, phosphotransacetylase and acylating acetaldehyde dehydrogenase making up the "phosphoketolase pathway." Strain A2 produces significantly higher level of acetate than the wild-type precursor strain FERMAXGOLD^{®}. The reasons for elevated acetate production in strain A2 are, firstly, that acetyl-phosphate, a product of phosphoketolase reaction, is chemically unstable and can undergo spontaneous hydrolysis into acetic and phosphoric acids and, secondly, acetyl-CoA formed in the phosphoketolase pathway reduces the metabolic demand for synthesizing it from acetic acid (the metabolic pathway yeast normally uses for production of cytosolic Ac-CoA anaerobically).

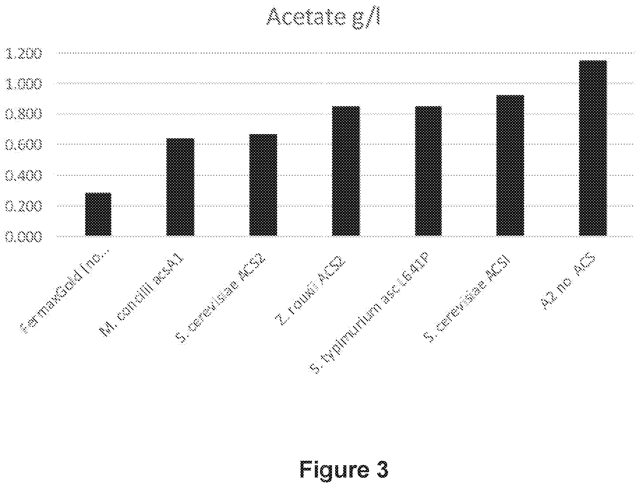

Strain A2, which is an uracil auxotroph, was transformed to uracil prototrophy with a series of five vectors directing the expression of five different ACS. Except for the ACS coding sequences, the vectors were identical. The prototype vector is pX(DeltaTDH_ACSSalty) expressing *S. typhimurium* acs L641P variant (Figure 1). In all cases the large *Swa*I fragment of the respective vector was used as the transforming DNA (including all the functional yeast elements but excluding the sequences needed for propagation of the vector in bacteria). Eight, randomly-selected transformants from each of the five transformations were selected and cultivated in a medium containing 60 g/l glucose, 0.67 g/l yeast nitrogen base without amino acids and ammonium sulfate, 2 g/l urea. The cultivations were performed for 48 hours at 32°C with shaking. The culture media was then analyzed for fermentation products by HPLC. The results of these experiments are summarized in Table 1 and the graph in Figure 3.

Surprisingly, the over-expression of *ACS*2 from *S. cerevisiae,* encoding the "low affinity" ACS, efficiently reduces acetate accumulation in culture medium. Such observations contradict published literature describing that over-expression of either nor *ACS*2 does not have an appreciable effect on acetate accumulation in yeast cultures (de Jong-Gubbels et al. (1998) FEMS Microbiology Letters 165:15-20). *ACS*2 from osmophilic yeast, *Z*. *rouxii,* was less efficient than its counterpart from *S. cerevisiae* but still performed slightly better than "industry standard" ACS previously described as useful for reduced acetate production in yeast (*i.e.,* a L641P mutant form of ACS from *S. typhimurium*). The overall winner of the study was the enzyme encoded by *acsA*1 gene of *M. concilii,* an archaeal microorganism from soil known to utilize acetate as the preferred carbon source.

**Table 1. Acetate production by high acetate strain A2 and its ACS-expressing derivatives**

| **Strain** | **Acetate (g/L)** |
|---|---|
| | Average of 8 cultures |
| FERMAXGOLD^{®} (no PKL pathway) | 0.286 |
| A2 expressing *Methanosaeta concilii* acsA1 | 0.641 |
| A2 expressing *S. cerevisiae ACS2* | 0.666 |
| A2 expressing *Z*. *rouxii ACS2* | 0.849 |
| A2 expressing *S. typimurium asc* L641P | 0.852 |
| A2 expressing *S. cerevisiae ACSI* | 0.925 |
| A2 no ACS | 1.150 |

### SEQUENCES

Polyucleotide sequence of the *ACS1* gen*e* from *S. cerevisiae* strain FERMAXGOLD^{®} (SEQ ID NO: 1): 
ATGTCGCCCTCTGCCGTACAATCATCAAAACTAGAAGAACAGTCAAGTGAAATTGACAAGTTG
AAAGCAAAAATGTCCCAGTCTGCCTCCACTGCGCAGCAGAAGAAGGAACATGAGTATGAACAT
TTGACCTCGGTCAAGATCGTGCCACAACGGCCCATCTCAGATAGACTGCAGCCCGCAATTGCT
ACCCACTATTCTCCACACTTGGACGGGTTGCAGGACTATCAGCGCTTGCACAAGGAGTCTATT
GAAGACCCTGCTAAGTTCTTCGGTTCTAAAGCTACCCAATTTTTAAACTGGTCTAAGCCATTC
GATAAGGTGTTCATCCCAGACCCTAAAACGGGCAGGCCCTCCTTCCAGAACAATGCATGGTTC
CTCAACGGCCAATTAAACGCCTGTTACAACTGTGTTGACAGACATGCCTTGAAGACCCCTAAC
AAGAAAGCCATTATTTTCGAAGGTGACGAGCCTGGCCAAGGCTATTCCATTACCTACAAGGAA
CTACTTGAAGAAGTTTGTCAAGTGGCACAAGTGCTGACTTACTCTATGGGCGTTCGCAAGGGC
GATACTGTTGCCGTGTACATGCCTATGGTCCCAGAAGCAATCATAACCTTGTTGGCCATTTCC
CGTATCGGTGCCATTCACTCCGTAGTCTTTGCCGGGTTTTCTTCCAACTCCTTGAGAGATCGT
ATCAACGATGGGGACTCTAAAGTTGTCATCACTACAGATGAATCCAACAGAGGTGGTAAAGTC
ATTGAGACTAAAAGAATTGTTGATGACGCGCTAAGAGAGACCCCAGGCGTGAGACACGTCTTG
GTTTATAGAAAGACCAACAATCCATCTGTTGCTTTCCATGCCCCCAGAGATTTGGATTGGGCA
 
ACAGAAAAGAAGAAATACAAGACCTACTATCCATGCACACCCGTTGATTCTGAGGATCCATTA
TTCTTGTTGTATACGTCTGGTTCTACTGGTGCCCCCAAGGGTGTTCAACATTCTACCGCAGGT
TACTTGCTGGGAGCTTTGTTGACCATGCGCTACACTTTTGACACTCACCAAGAAGACGTTTTC
TTCACAGCTGGAGACATTGGCTGGATTACAGGCCACACTTATGTGGTTTATGGTCCCTTACTA
TATGGTTGTGCCACTTTGGTCTTTGAAGGACGCCTGCATACCCAAATTACTCCCGTTATTGG
GATATTATTGATGAACACAAAGTCACCCAATTTTATGTTGCGCCAACTGCTTTGCGTTTGTTG
AAAAGAGCTGGTGATTCCTACATCGAAAATCATTCCTTAAAATCTTTGCGTTGCTTGGGTTCG
GTCGGTGAGCCAATTGCTGCTGAAGTTTGGGAGTGGTACTCTGAAAAAATAGGTAAAAATGAA
ATCCCCATTGTAGACACCTACTGGCAAACAGAATCTGGTTCGCATCTGGTCACCCCGCTGGCT
GGTGGTGTCACACCAATGAAACCGGGTTCTGCCTCATTCCCCTTCTTCGGTATTGATGCAGTT
GTTCTTGACCCTAACACTGGTGAAGAACTTAACACCAGCCACGCAGAGGGTGTCCTTGCCGTC
AAAGCTGCATGGCCATCATTTGCAAGAACTATTTGGAAAAATCATGATAGGTATCTAGACACT
TATTTGAACCCTTACCCTGGCTACTATTTCACTGGTGATGGTGCTGCAAAGGATAAGGATGGT
TATATCTGGATTTTGGGTCGTGTAGACGATGTGGTGAACGTCTCTGGTCACCGTCTGTCTACC
GCTGAAATTGAGGCTGCTATTATCGAAGATCCAATTGTGGCCGAGTGTGCTGTTGTCGGATTC
AACGATGACTTGACTGGTCAAGCAGTTGCTGCATTTGTGGTGTTGAAAAACAAATCTAGTTGG
TCCACCGCAACAGATGATGAATTACAAGATATCAAGAAGCATTTGGTCTTTACTGTTAGAAAA
GACATCGGGCCATTTGCCGCACCAAAATTGATCATTTTAGTGGATGACTTGCCCAAGACAAGA
TCTGGCAAAATTATGAGACGTATTTTAAGAAAAATCCTAGCAGGAGAAAGTGACCAACTAGGC
GACGTTTCTACATTGTCAAACCCTGGCATTGTTAGACATCTAATTGATTCGGTCAAGTTGTAA
Amino acid sequence of the ACS1p from *S. cerevisiae* strain FERMAXGOLD^{®} (SEQ ID NO: 2): 
MSPSAVQSSKLEEQSSEIDKLKAKMSQSASTAQQKKEHEYEHLTSVKIVPQRPISDRLQPAIAT
HYSPHLDGLQDYQRLHKESIEDPAKFFGSKATQFLNWSKPFDKVFIPDPKTGRPSFQNNAWFLN
GQLNACYNCVDRHALKTPNKKAIIFEGDEPGQGYSITYKELLEEVCQVAQVLTYSMGVRKGDTV
AVYMPMVPEAIITLLAISRIGAIHSVVFAGFSSNSLRDRINDGDSKVVITTDESNRGGKVIETK
RIVDDALRETPGVRHVLVYRKTNNPSVAFHAPRDLDWATEKKKYKTYYPCTPVDSEDPLFLLYT
SGSTGAPKGVQHSTAGYLLGALLTMRYTFDTHQEDVFFTAGDIGWITGHTYVVYGPLLYGCATL
VFEGTPAYPNYSRYWDIIDEHKVTQFYVAPTALRLLKRAGDSYIENHSLKSLRCLGSVGEPIAA
EVWEWYSEKIGKNEIPIVDTYWQTESGSHLVTPLAGGVTPMKPGSASFPFFGIDAVVLDPNTGE
 
ELNTSHAEGVLAVKAAWPSFARTIWKNHDRYLDTYLNPYPGYYFTGDGAAKDKDGYIWILGRVD
DVVNVSGHRLSTAEIEAAIIEDPIVAECAVVGFNDDLTGQAVAAFVVLKNKSSWSTATDDELQD
IKKHLVFTVRKDIGPFAAPKLIILVDDLPKTRSGKIMRRILRKILAGESDQLGDVSTLSNPGIV
RHLIDSVKL
Polynucleotide sequence of the *ACS2* gene from *S. cerevisiae* strain FERMAXGOLD^{®} (SEQ ID NO: 3): 
ATGACAATCAAGGAACATAAAGTAGTTCATGAAGCTCACAACGTAAAGGCTCTTAAGGCTCCTC
AACATTTTTACAACAGCCAACCCGGCAAGGGTTACGTTACTGATATGCAACATTATCAAGAAAT
GTATCAACAATCTATCAATGAGCCAGAAAAATTCTTTGATAAGATGGCTAAGGAATACTTGCAT
TGGGATGCTCCATACACCAAAGTTCAATCTGGTTCATTGAACAATGGTGATGTTGCATGGTTTT
TGAACGGTAAATTGAATGCATCATACAATTGTGTTGACAGACATGCCTTTGCTAATCCCGACAA
GCCAGCTTTGATCTATGAAGCTGATGACGAATCCGACAACAAAATCATCACATTTGGTGAATTA
CTCAGAAAAGTTTCCCAAATCGCTGGTGTCTTAAAAAGCTGGGGCGTTAAGAAAGGTGACACAG
TGGCTATCTATTTGCCAATGATTCCAGAAGCGGTCATTGCTATGTTGGCTGTGGCTCGTATTGG
TGCTATTCACTCTGTTGTCTTTGCTGGGTTCTCCGCTGGTTCGTTGAAAGATCGTGTCGTTGAC
GCTAATTCTAAAGTGGTCATCACTTGTGATGAAGGTAAAAGAGGTGGTAAGACCATCAACACTA
AAAAAATTGTTGACGAAGGTTTGAACGGAGTCGATTTGGTTTCCCGTATCTTGGTTTTCCAAAG
AACTGGTACTGAAGGTATTCCAATGAAGGCCGGTAGAGATTACTGGTGGCATGAGGAGGCCGCT
AAGCAGAGAACTTACCTACCTCCTGTTTCATGTGACGCTGAAGATCCTCTATTTTTGTTATACA
CTTCCGGTTCCACTGGTTCTCCAAAGGGTGTCGTTCACACTACAGGTGGTTATTTATTAGGTGC
CGCTTTAACAACTAGATACGTTTTTGATATTCACCCAGAAGATGTTCTCTTCACTGCCGGTGAC
GTCGGCTGGATCACGGGTCACACCTATGCTCTATATGGTCCATTAACCTTGGGTACCGCCTCAA
TAATTTTCGAATCCACTCCTGCCTACCCAGATTATGGTAGATATTGGAGAATTATCCAACGTCA
CAAGGCTACCCATTTCTATGTGGCTCCAACTGCTTTAAGATTAATCAAACGTGTAGGTGAAGCC
GAAATTGCCAAATATGACACTTCCTCATTACGTGTCTTGGGTTCCGTCGGTGAACCAATCTCTC
CAGACTTATGGGAATGGTATCATGAAAAAGTGGGTAACAAAAACTGTGTCATTTGTGACACTAT
GTGGCAAACAGAGTCTGGTTCTCATTTAATTGCTCCTTTGGCAGGTGCTGTCCCAACAAAACCT
GGTTCTGCTACCGTGCCATTCTTTGGTATTAACGCTTGTATCATTGACCCTGTTACAGGTGTGG
AATTAGAAGGTAATGATGTCGAAGGTGTCCTTGCCGTTAAATCACCATGGCCATCAATGGCTAG
ATCTGTTTGGAACCACCACGACCGTTACATGGATACTTACTTGAAACCTTATCCTGGTCACTAT
TTCACAGGTGATGGTGCTGGTAGAGATCATGATGGTTACTACTGGATCAGGGGTAGAGTTGACG
 
ACGTTGTAAATGTTTCCGGTCATAGATTATCCACATCAGAAATTGAAGCATCCATCTCAAATCA
CGAAAACGTCTCGGAAGCTGCTGTTGTCGGTATTCCAGATGAATTGACCGGTCAAACCGTCGTT
GCATATGTTTCCCTAAAAGATGGTTATCTACAAAACAACGCTACTGAAGGTGATGCAGAACACA
TCACACCAGATAATTTACGTAGAGAATTGATCTTACAAGTTAGGGGTGAGATTGGTCCTTTCGC
CTCACCAAAAACCATTATTCTAGTTAGAGATCTACCAAGAACAAGGTCAGGAAAGATTATGAGA
AGAGTTCTAAGAAAGGTTGCTTCTAACGAAGCCGAACAGCTAGGTGACCTAACTACTTTGGCCA
ACCCAGAAGTTGTACCTGCCATCATTTCTGCTGTAGAGAACCAATTTTCTCTCAAAAAAAGAA
ATAA
Amino acid sequence of the ACS2p from *S. cerevisiae* strain FERMAXGOLD^{®} (SEQ ID NO: 4): 
MTIKEHKVVHEAHNVKALKAPQHFYNSQPGKGYVTDMQHYQEMYQQSINEPEKFFDKMAKEYLH
WDAPYTKVQSGSLNNGDVAWFLNGKLNASYNCVDRHAFANPDKPALIYEADDESDNKIITFGEL
LRKVSQIAGVLKSWGVKKGDTVAIYLPMIPEAVIAMLAVARIGAIHSVVFAGFSAGSLKDRVVD
ANSKVVITCDEGKRGGKTINTKKIVDEGLNGVDLVSRILVFQRTGTEGIPMKAGRDYWWHEEAA
KQRTYLPPVSCDAEDPLFLLYTSGSTGSPKGVVHTTGGYLLGAALTTRYVFDIHPEDVLFTAGD
VGWITGHTYALYGPLTLGTASIIFESTPAYPDYGRYWRIIQRHKATHFYVAPTALRLIKRVGEA
EIAKYDTSSLRVLGSVGEPISPDLWEWYHEKVGNKNCVICDTMWQTESGSHLIAPLAGAVPTKP
GSATVPFFGINACIIDPVTGVELEGNDVEGVLAVKSPWPSMARSVWNHHDRYMDTYLKPYPGHY
FTGDGAGRDHDGYYWIRGRVDDVVNVSGHRLSTSEIEASISNHENVSEAAVVGIPDELTGQTVV
AYVSLKDGYLQNNATEGDAEHITPDNLRRELILQVRGEIGPFASPKTIILVRDLPRTRSGKIMR
RVLRKVASNEAEQLGDLTTLANPEVVPAIISAVENQFFSQKKK
Nucleotide sequence of the *ACS2* gene from Z. *rouxii* strain CBS762 (SEQ ID NO: 5) 
ATGACAACTAAGGAACATAAGACCGTTCATGAGGATAAACCATTTACAAAGACGAAGTTTGGTT
CATTGGAAAATGGTGATACTACTTGGTTTTAAACGGTGAGTTGAATGCTGCTTACAACTGTGT
TGATAGACATGCTTTTGCCAATCCTGATAAACCAGCATTGATCTACGAAGCGGATGAAGAAGCA
GACAACAGGGTTGTTACATTCGGTGAGCTTTTGAGACAAGTTTCTCAAGTTGCTGGTGTTCTTC
ACAGCTGGGGGGTTAGAAAAGGTGATACCGTCGCAGTTTATCTACCAATGATTCCTGAAGCTGT
TGTTGCAATGTTGGCCGTTGCCAGATTAGGTGCAATTCATTCTGTTGTTTTTGCAGGTTTTTCA
GCAGGTTCCTTGAAGGATCGTGTTGTAGACGCAGGTTGTAAAGTTGTTATTACTTGTGATCAAG
GTAAAAGAGGTAGCAAAACCGTTCATACAAAGAAAATTGTCGATGAAGGTTTAAATGGAATTTC
TCAAGTTTCTCATATTCTTGTCTTCCAAAGGACAGGTGCTGAAGGATCCCAATGACACCTGGC
 
AGAGATTACTGGTGGCACGAAGAAGCTGCTAAGCAAAGAGGTTACATTCCACCAGTTCCTTGTA
GTGCTGAAGATCCATTATTCCTCTTGTACACTTCAGGTTCCACCGGGTCACCAAAGGGTGTGGT
CCATTCAACCGGTGGTTATCTATTGGGTGCAGCCATGACCACTAGATATGTGTTTGACATCCAT
CCAGAAGATGTCTTATTCACAGCAGGTGATGTTGGTTGGATTACAGGTCATACTTATGCTCTTT
ATGGTCCATTAGCGCTAGGTACTGCATCAATCATCTTTGAATCAACACCAGCTTATCCTGATTA
CGGTAGATATTGGAGAATCATTCAGCGTCATAAGGCAACTCATTTCTACGTGGCTCCAACAGCC
ATGAGATTGATTAAGCGTGTAGGTGAGGCTGAAATTCCAAAATACGATCTATCTTCACTAAGAG
TTCTTGGATCAGTCGGTGAACCAATTTCACCAGATCTTTGGGAATGGTACAACGAAAAAATTGG
TCACAACAACTGTGTCGTTTGTGATACCATGTGGCAAACCGAATCTGGTTCTCATTTAATTGCT
CCATTAGCAGGTGCAGTCCCAACAAAACCTGGTTCCGCTACAGTTCCATTCTTTGGTGTTGATG
CTTGTATCATTGACCCAGTCACTGGTGTTGAATTACAAGGTAATGACGTGGAAGGTGTTTTGGC
AGTAAAATCATCTTGGCCATCTATGGCAAGATCAGTTTGGCAAAATCACAACCGTTTCCAGGAG
ACTTACTTGCAACCATACCCTGGTTACTACTTTACAGGTGATGGTGCAGGTAGAGACCATGATG
GTTACTACTGGATCAGAGGTAGAGTTGATGATGTGGTTAACGTTTCTGGCCACAGATTGTCCAC
TGCTGAAATTGAAGCTTCATTGACCAACCATGATAATGTTTCTGAATCTGCTGTCGTAGGTATT
CCTGACGAATTGACCGGTCAAACCGTCATTGCCTTCGTTGCATTGAAAGATGGTACTCCAAGTC
AAGGTGATGCGAGTGCTAACGTTCGTCGTGAATTGGTGCTCCAAGTTAGAGGTGAAATTGGTCC
ATTTGCTGCTCCTAAATGTGTCATCTTGGTTAAAGATCTGCCAAAGACTAGATCCGGTAAAATC
ATGAGAAGAGTCCTAAGAAAAGTTGCATCTAATGAAGCTGATCAACTGGGTGATCTATCTACTA
TGGCTAACGCTGAAGTCGTTCCAGGTATCATTGCAGCTGTTGATGAACAATATTTTGCTGAGAA
GAAGAAATAA
Amino acid sequence of the ACS2p from *Z*. *rouxii* strain CBS762 (SEQ ID NO: 6): 
MTTKEHKTVHEAQNVVARHAPEHFYKSQPGLGYVKDMKQYQEMYKQSVEDPETFFGTKAQELLH
WDKPFTKTKFGSLENGDTTWFLNGELNAAYNCVDRHAFANPDKPALIYEADEEADNRVVTFGEL
LRQVSQVAGVLHSWGVRKGDTVAVYLPMIPEAVVAMLAVARLGAIHSVVFAGFSAGSLKDRVVD
AGCKVVITCDQGKRGSKTVHTKKIVDEGLNGISQVSHILVFQRTGAEGIPMTPGRDYWWHEEAA
KQRGYIPPVPCSAEDPLFLLYTSGSTGSPKGVVHSTGGYLLGAAMTTRYVFDIHPEDVLFTAGD
VGWITGHTYALYGPLALGTASIIFESTPAYPDYGRYWRIIQRHKATHFYVAPTAMRLIKRVGEA
EIPKYDLSSLRVLGSVGEPISPDLWEWYNEKIGHNNCVVCDTMWQTESGSHLIAPLAGAVPTKP
GSATVPFFGVDACIIDPVTGVELQGNDVEGVLAVKSSWPSMARSVWQNHNRFQETYLQPYPGYY
FTGDGAGRDHDGYYWIRGRVDDVVNVSGHRLSTAEIEASLTNHDNVSESAVVGIPDELTGQTVI
 
AFVALKDGTPSQGDASANVRRELVLQVRGEIGPFAAPKCVILVKDLPKTRSGKIMRRVLRKVAS
NEADQLGDLSTMANAEVVPGIIAAVDEQYFAEKKK
Synthetic, yeast adopted DNA sequence encoding *Salmonella typhimurium* ACS L641P mutant (SEQ ID NO: 7): 
ATGAACCAACAAGACATAGAACAAGTAGTAAAAGCCGTATTATTAAAGATGAAAGACTCCTCTC
AACCAGCCTCAACCGTACACGAAATGGGTGTTTTTGCCTCTTTGGATGACGCTGTCGCTGCAGC
CAAAAGAGCCCAACAAGGTTTGAAGTCAGTTGCTATGAGACAATTAGCAATCCATGCCATTAGA
GAAGCAGGTGAAAAACACGCCAGAGAATTGGCTGAATTAGCAGTATCCGAAACTGGTATGGGTA
GAGTTGATGACAAATTCGCTAAGAATGTCGCTCAAGCAAGAGGTACACCAGGTGTCGAATGTTT
GAGTCCTCAAGTATTAACAGGTGACAATGGTTTGACCTTAATTGAAAACGCCCCATGGGGTGTT
GTCGCTTCTGTTACACCATCAACCAATCCTGCTGCAACTGTTATAAATAACGCAATCTCTTTGA
TCGCCGCTGGTAACTCAGTAGTTTTTGCTCCACATCCTGCAGCCAAAAAGGTTTCCCAAAGAGC
AATTACATTGTTAAATCAAGCCGTCGTAGCTGCAGGTGGTCCAGAAAATTTGTTAGTAACCGTT
GCTAACCCTGATATCGAAACTGCACAAAGATTATTCAAGTATCCAGGTATCGGTTTGTTAGTTG
TCACAGGTGGTGAAGCTGTAGTTGATGCCGCTAGAAAACACACCAATAAGAGATTGATTGCAGC
CGGTGCAGGTAACCCACCTGTCGTAGTTGATGAAACTGCTGACTTACCAAGAGCTGCACAATCC
ATCGTTAAGGGTGCAAGTTTCGATAACAACATCATCTGCGCTGACGAAAAGGTTTTAATTGTCG
TAGATTCTGTCGCTGACGAATTGATGAGATTAATGGAAGGTCAACATGCAGTTAAATTGACAGC
CGCTCAAGCCGAACAATTGCAACCAGTTTTGTTGAAAAATATAGATGAACGTGGTAAAGGTACC
GTATCAAGAGATTGGGTTGGTAGAGACGCAGGTAAAATTGCAGCCGCTATAGGTTTGAACGTTC
CTGATCAAACTAGATTGTTGTTCGTTGAAACACCAGCTAACCATCCTTTCGCAGTAACAGAAAT
GATGATGCCAGTTTTACCTGTTGTCAGAGTTGCTAATGTCGAAGAAGCCATAGCTTTGGCAGTT
CAATTAGAAGGTGGTTGTCATCACACCGCAGCCATGCACTCCAGAAATATCGATAATATGAACC
AAATGGCCAACGCTATCGACACTTCTATTTTCGTTAAAAACGGTCCATGCATTGCTGGTTTGGG
TTTAGGTGGTGAAGGTTGGACTACAATGACCATAACCACTCCTACTGGTGAAGGTGTCACTTCT
GCAAGAACATTTGTAAGATTGAGAAGATGTGTCTTAGTAGATGCTTTCAGAATTGTTTAG
Amino acid sequence of the *Salmonella typhimurium* ACS L641P mutant (SEQ ID NO: 8): 
MSQTHKHAIPANIADRCLINPEQYETKYKQSINDPDTFWGEQGKILDWITPYQKVKNTSFAPGN
VSIKWYEDGTLNLAANCLDRHLQENGDRTAIIWEGDDTSQSKHISYRELHRDVCRFANTLLDLG
IKKGDVVAIYMPMVPEAAVAMLACARIGAVHSVIFGGFSPEAVAGRIIDSSSRLVITADEGVRA
 
GRSIPLKKNVDDALKNPNVTSVEHVIVLKRTGSDIDWQEGRDLWWRDLIEKASPEHQPEAMNAE
DPLFILYTSGSTGKPKGVLHTTGGYLVYAATTFKYVFDYHPGDIYWCTADVGWVTGHSYLLYGP
LACGATTLMFEGVPNWPTPARMCQVVDKHQVNILYTAPTAIRALMAEGDKAIEGTDRSSLRILG
SVGEPINPEAWEWYWKKIGKEKCPVVDTWWQTETGGFMITPLPGAIELKAGSATRPFFGVQPAL
VDNEGHPQEGATEGNLVITDSWPGQARTLFGDHERFEQTYFSTFKNMYFSGDGARRDEDGYYWI
TGRVDDVLNVSGHRLGTAEIESALVAHPKIAEAAVVGIPHAIKGQAIYAYVTLNHGEEPSPELY
AEVRNWVRKEIGPLATPDVLHWTDSLPKTRSGKIMRRILRKIAAGDTSNLGDTSTLADPGVVEK
PLEEKQAIAMPS
Synthetic, yeast adopted DNA sequence encoding *Methanosaeta concilii* acsA1p (SEQ ID NO: 9): 
ATGTTGAAGTTGGCCGGTAAAGAAGATAAGAAGTTGAAAACCACTGTTTTCCAAGACGAAACCA
GAATTTTCAACCCACCAAAAGAATTGGTCGAAAAGTCCATAGTTATGCAATGGATGAAGAAGAA
GGGTTTCAAGACCGAAAAAGAAATGAGAGCTTGGTGTTCCTCTGATGAACACTATTTGGAATTT
TGGGACGAAATGGCTAAGACCTATGTTGATTGGCATAAGACTTACACCAAGGTTATGGATGATT
CCGAAATGCCATACTTCCATTGGTTTACTGGTGGTGAAATCAACATTACCTACAACGCTGTTGA
TAGACATGCTAAAGGTGCTAAGAAAGATAAGGTTGCCTACATCTGGATTCCAGAACCTACTGAT
CAACCAGTTCAAAAGATTACTTACGGTGACTTGTACAAAGAAGTCAACAAGTTTGCTAACGGTT
TGAAGTCTTTGGGTTTGAAAAAGGGTGACAGAGTCTCTATCTACATGCCAATGATTCCACAATT
GCCAATTGCTATGTTGGCTTGTGCTAAGTTGGGTGTTATTCACTCTGTTGTTTTCTCCGGTTTT
TCCAGTAAAGGTTTGATGGATAGAGCTGCTGATTGTGGTTCAAGAGCTATTATTACTGTTGACG
GTTTCTACAGAAGAGGTAAACCAGTTCCATTGAAGCCAAATGCTGATGAAGCTGCTGGTGGTGC
TCCATCTGTTGAAAAGATTATCGTTTACAAAAGAGCCGGTGTCGATGTCTCTATGAAGGAAGGT
AGAGATGTTTGGTGGCATGATTTGGTTAAGGGTCAATCTGAAGAATGTGAACCAGTTTGGGTTG
ATCCAGAACATAGATTGTACATCTTGTACACCTCTGGTACTACTGGTAAGCCAAAAGGTATTGA
ACATGCAACTGGTGGTAATGCTGTTGGTCCAGCTCAAACTTTACATTGGGTTTTCGATTTGAAG
GATGATGATGTATGGTGGTGTACTGCTGATATTGGTTGGGTTACTGGTCATTCTTACATCGTTT
ATGCCCCATTGATTTTGGGTATGACCTCTTTGATGTATGAAGGTGCTGCAGATTATCCAGATTT
TGGTAGATGGTGGAAGAACATCCAAGATCATAAGGTTACTGTCTTGTATACTGCTCCAACTGCT
GTTAGAATGTTCATGAAGCAAGGTGCTGAATGGCCAGATAAGTATGATTTGTCCTCCTTGAGAT
TATTGGGTTCTGTTGGTGAACCTATTAACCCTGAAGCCTGGATGTGGTATAGAGAACATATTGG
TAGAGGTGAATTGCAAATCATGGATACTTGGTGGCAAACTGAAACCGGTACTTTTTTGAACTCT
 
CCATTGCCTATTACCCCATTGAAACCAGGTTCTTGTACTTTTCCATTGCCAGGTTACGATATCT
CCATTTTGGACGAAGAAGGTAACGAAGTTCCATTAGGTTCAGGTGGTAATATCGTTGCTTTGAA
ACCATACCCATCTATGTTGAGAGCTTTTTGGGGTGACAAAGAAAGATTCATGAAGGAATACTGG
CAATTCTACTGGGATGTTCCAGGTAGAAGAGGTGTTTATTTGGCTGGTGATAAGGCTCAAAGAG
ATAAGGACGGTTACTTCTTCATTCAAGGTAGAATCGATGATGTTTTGTCCGTTGCTGGTCATAG
AATTGCTAATGCTGAAGTTGAATCTGCTTTGGTTGCTCATCCAAAAATTGCTGAAGCTGCAGTT
GTTGGTAAACCTGATGAAGTAAAAGGTGAATCTATCGTTGCCTTCGTTATCTTGAGAGTTGGTA
ATGAACCATCTCCAGAATTGGCTAAAGATGCCATTGCTTTCGTTAGAAAAACTTTGGGTCCAGT
TGCTGCTCCTACTGAAGTTCATTTTGTTAACGATTTGCCAAAGACTAGATCCGGTAAGATCATG
AGAAGAGTTGTTAAGGCTAGAGCTTTGGGTAATCCAGTTGGTGATATTTCCACTTTGATGAATC
CTGAAGCCGTTGATGGTATTCCAAAGATCGTTTAA
Amino acid sequence of the *Methanosaeta concilii* acsA1p (SEQ ID NO: 10):

MLKLAGKEDKKLKTTVFQDETRIFNPPKELVEKSIVMQWMKKKGFKTEKEMRAWCSSDEHYLEF
WDEMAKTYVDWHKTYTKVMDDSEMPYFHWFTGGEINITYNAVDRHAKGAKKDKVAYIWIPEPTD
QPVQKITYGDLYKEVNKFANGLKSLGLKKGDRVSIYMPMIPQLPIAMLACAKLGVIHSVVFSGF
SSKGLMDRAADCGSRAIITVDGFYRRGKPVPLKPNADEAAGGAPSVEKIIVYKRAGVDVSMKEG
RDVWWHDLVKGQSEECEPVWVDPEHRLYILYTSGTTGKPKGIEHATGGNAVGPAQTLHWVFDLK
DDDVWWCTADIGWVTGHSYIVYAPLILGMTSLMYEGAADYPDFGRWWKNIQDHKVTVLYTAPTA
VRMFMKQGAEWPDKYDLSSLRLLGSVGEPINPEAWMWYREHIGRGELQIMDTWWQTETGTFLNS
PLPITPLKPGSCTFPLPGYDISILDEEGNEVPLGSGGNIVALKPYPSMLRAFWGDKERFMKEYW
QFYWDVPGRRGVYLAGDKAQRDKDGYFFIQGRIDDVLSVAGHRIANAEVESALVAHPKIAEAAV
VGKPDEVKGESIVAFVILRVGNEPSPELAKDAIAFVRKTLGPVAAPTEVHFVNDLPKTRSGKIM
RRVVKARALGNPVGDISTLMNPEAVDGIPKIV

## Claims

1. Modified yeast cells, comprising an exogenous gene encoding a protein having at least 80% amino acid sequence identity to a polypeptide of SEQ ID NO: 10, or a fragment thereof, the protein and fragment thereof having acetyl CoA synthetase (ACS) activity,
wherein the modified cells produce a reduced amount of acetate and/or grow in the presence of an increased amount of acetate, compared to otherwise identical yeast cells not comprising the exogenous gene.

2. The modified yeast cells of claim 1, further comprising a genetic modification that, in the absence of the exogenous gene, causes the yeast cells to produce an increased amount of acetate.

3. The modified yeast cells of claim 2, wherein the genetic modification is the introduction of one or more genes of the phosphoketolase pathway or the deletion or disruption of genes of the glycerol synthesis pathway.

4. The modified yeast cells of any of the preceding claims, wherein the reduction in the amount of acetate produced is at least 20%, at least 30% or at least 40%.

5. The modified yeast cells of any of the preceding claims, wherein the protein has at least 90%, at least 95% or at least 97% amino acid sequence identity to a polypeptide of SEQ ID NO: 10, or a fragment thereof, the protein and fragment thereof having ACS activity.

6. The modified yeast cells of any of the preceding claims, further comprising a gene encoding a carbohydrate processing enzyme or other protein of interest.

7. A method for reducing the amount of acetate produced by yeast cells during a fermentation of a starch hydrolysate, comprising, introducing into the yeast an exogenous gene encoding a protein having at least 80% amino acid sequence identity to a polypeptide of SEQ ID NO: 10, or a fragment thereof, the protein and fragment thereof having ACS activity, in order to produce the modified yeast cells of any of claims 1-6, and using the modified yeast cells in the fermentation of a starch hydrolysate.

8. The method of claim 7, wherein the yeast cells further comprise a genetic modification that, in the absence of the exogenous gene, would cause the yeast cells to produce an increased amount of acetate.

9. The method of claim 8, wherein the genetic modification is the introduction of genes of the phosphoketolase pathway or the deletion or disruption of genes of the glycerol synthesis pathway.

10. The method of any of claims 7-9, wherein the reduction in the amount of acetate produced is at least 20%, at least 30% or at least 40%.

11. The method of any of claims 7-10, wherein the protein has at least 90%, at least 95% or at least 97% amino acid sequence identity to a polypeptide of SEQ ID NO: 10, or a fragment thereof, the protein and fragment thereof having ACS activity.

## Patentansprüche

1. Modifizierte Hefezellen, umfassend ein exogenes Gen, das ein Protein mit mindestens 80 % Aminosäuresequenzidentität zu einem Polypeptid unter SEQ ID NO: 10 oder einem Fragment davon codiert, wobei das Protein und Fragment davon Acetyl-CoA-Synthetase(ACS)-Aktivität aufweisen,
wobei die modifizierten Zellen eine reduzierte Menge von Acetat herstellen und/oder in Gegenwart einer erhöhten Menge von Acetat wachsen, verglichen mit ansonsten identischen Hefezellen, die das exogene Gen nicht umfassen.

2. Modifizierte Hefezellen nach Anspruch 1, ferner umfassend eine genetische Modifikation, die in Abwesenheit des exogenen Gens die Hefezellen dazu bringt eine erhöhte Menge von Acetat herzustellen.

3. Modifizierte Hefezellen nach Anspruch 2, wobei es sich bei der genetischen Modifikation um die Einführung eines oder mehrerer Gene des Phosphoketolase-Wegs oder die Deletion oder Unterbrechung von Genen des Glycerolsynthese-Wegs handelt.

4. Modifizierte Hefezellen nach einem der vorhergehenden Ansprüche, wobei die Reduzierung in der Menge von hergestelltem Acetat mindestens 20 %, mindestens 30 % oder mindestens 40 % beträgt.

5. Modifizierte Hefezellen nach einem der vorhergehenden Ansprüche, wobei das Protein mindestens 90 %, mindestens 95 % oder mindestens 97 % Aminosäuresequenzidentität zu einem Polypeptid unter SEQ ID NO: 10 oder einem Fragment davon aufweist, wobei das Protein und Fragment davon ACS-Aktivität aufweisen.

6. Modifizierte Hefezellen nach einem der vorhergehenden Ansprüche, ferner umfassend ein Gen, das ein kohlenhydratverarbeitendes Enzym oder anderes Protein von Interesse codiert.

7. Verfahren zum Reduzieren der Menge von Acetat, die von Hefezellen während einer Fermentation eines Stärkehydrolysats hergestellt wird, umfassend Einführen eines exogenen Gens in die Hefe, das ein Protein mit mindestens 80 % Aminosäuresequenzidentität zu einem Polypeptid unter SEQ ID NO: 10 oder einem Fragment davon codiert, wobei das Protein und Fragment davon ACS-Aktivität aufweisen, um die modifizierten Hefezellen nach einem der Ansprüche 1-6 herzustellen und zum Verwenden der modifizierten Hefezellen bei der Fermentation eines Stärkehydrolysats.

8. Verfahren nach Anspruch 7, wobei die Hefezellen ferner eine genetische Modifikation umfassen, die in Abwesenheit des exogenen Gens die Hefezellen dazu bringen würde eine erhöhte Menge von Acetat herzustellen.

9. Verfahren nach Anspruch 8, wobei es sich bei der genetischen Modifikation um die Einführung von Genen des Phosphoketolase-Wegs oder die Deletion oder Unterbrechung von Genen des Glycerolsynthese-Wegs handelt.

10. Verfahren nach einem der Ansprüche 7-9, wobei die Reduzierung in der Menge von hergestelltem Acetat mindestens 20 %, mindestens 30 % oder mindestens 40 % beträgt.

11. Verfahren nach einem der Ansprüche 7-10, wobei das Protein mindestens 90 %, mindestens 95 % oder mindestens 97 % Aminosäuresequenzidentität zu einem Polypeptid unter SEQ ID NO: 10 oder einem Fragment davon aufweist, wobei das Protein und Fragment davon ACS-Aktivität aufweisen.

## Revendications

1. Cellules de levure modifiées, comprenant un gène exogène codant pour une protéine ayant au moins 80 % d'identité de séquence d'acides aminés avec un polypeptide de SEQ ID NO: 10, ou un fragment de celle-ci, la protéine et un fragment de celle-ci ayant une activité d'acétyl-CoA synthétase (ACS),
dans lesquelles les cellules modifiées produisent une quantité réduite d'acétate et/ou croissent en présence d'une quantité accrue d'acétate, par rapport à des cellules de levure par ailleurs identiques ne comprenant pas le gène exogène.

2. Cellules de levure modifiées selon la revendication 1, comprenant en outre une modification génétique qui, en l'absence du gène exogène, amène les cellules de levure à produire une quantité accrue d'acétate.

3. Cellules de levure modifiées selon la revendication 2, dans lesquelles la modification génétique est l'introduction d'un ou plusieurs gènes de la voie de la phosphocétolase ou la délétion ou la perturbation de gènes de la voie de synthèse du glycérol.

4. Cellules de levure modifiées selon l'une quelconque des revendications précédentes, dans lesquelles la réduction de la quantité d'acétate produite est d'au moins 20 %, d'au moins 30 % ou d'au moins 40 %.

5. Cellules de levure modifiées selon l'une quelconque des revendications précédentes, dans lesquelles la protéine possède au moins 90 %, au moins 95 % ou au moins 97 % d'identité de séquence d'acides aminés avec un polypeptide de SEQ ID NO: 10, ou un fragment de celle-ci, la protéine et le fragment de celle-ci ayant une activité d'ACS.

6. Cellules de levure modifiées selon l'une quelconque des revendications précédentes, comprenant en outre un gène codant pour une enzyme de traitement de glucides ou une autre protéine d'intérêt.

7. Procédé de réduction de la quantité d'acétate produite par des cellules de levure lors d'une fermentation d'un hydrolysat d'amidon, comprenant, l'introduction dans la levure d'un gène exogène codant pour une protéine présentant au moins 80 % d'identité de séquence d'acides aminés avec un polypeptide de SEQ ID NO: 10, ou un fragment de celle-ci, la protéine et un fragment de celle-ci ayant une activité d'ACS, pour produire les cellules de levure modifiées selon l'une quelconque des revendications 1 à 6, et utilisant les cellules de levure modifiées dans la fermentation d'un hydrolysat d'amidon.

8. Procédé selon la revendication 7, dans lequel les cellules de levure comprennent en outre une modification génétique qui, en l'absence du gène exogène, amènerait les cellules de levure à produire une quantité accrue d'acétate.

9. Procédé selon la revendication 8, dans lequel la modification génétique est l'introduction de gènes de la voie de la phosphocétolase ou la délétion ou la perturbation de gènes de la voie de synthèse du glycérol.

10. Procédé selon l'une quelconque des revendications 7 à 9, dans lequel la réduction de la quantité d'acétate produite est d'au moins 20 %, d'au moins 30 % ou d'au moins 40 %.

11. Procédé selon l'une quelconque des revendications 7 à 10, dans lequel la protéine possède au moins 90 %, au moins 95 % ou au moins 97 % d'identité de séquence d'acides aminés avec un polypeptide de SEQ ID NO: 10, ou un fragment de celle-ci, la protéine et le fragment de celle-ci ayant une activité d'ACS.
